# EUROPEAN PATENT APPLICATION

(11) **EP 3 926 642 A1**
(43) Date of publication of application: **22.12.2021**
(21) Application number: 20180625.4
(22) Date of filing: 17.06.2020
(51) Int. Cl.: G16H 40/63, G16H 10/60

(54) **DYNAMIC POSITIONING FOR A VIRTUAL DISPLAY**

(71) Applicant: Koninklijke Philips N.V., 5656 AG Eindhoven (NL)
(72) Inventor: TALGORN, Elise Claude Valentine, 5656 AE Eindhoven (NL); DJAJADININGRAT, Johan Partomo, 5656 AE Eindhoven (NL); EERDEN, Jacco Christof, 5656 AE Eindhoven (NL)
(74) Representative: Philips Intellectual Property & Standards

(57) **Abstract**

Some embodiments are directed to a computer implemented method for positioning a virtual display for patient information. For example, a virtual display may be displayed for the viewer person at a display position to display the patient information on the virtual display. The display position may be determined by applying a rule base to a viewing context determined from a viewer identity and/or patient identity.

## Description

### FIELD OF THE INVENTION

The presently disclosed subject matter relates to a computer implemented method for positioning a virtual display, a computer readable medium, a system for positioning a virtual display.

### BACKGROUND OF THE INVENTION

In many health contexts there is a lot of information that has to be shared amongst several actors, e.g., amongst nurses, doctors, speech therapists, physiotherapists, patient and family members, etc. For example, information exchange may be needed in a hospital room such as, an intensive care unit (ICU), an operation room (OR), a treatment room, a ward, and so on.

For example, in the morning and evening, e.g., at the shift handover, nurses who end their shifts communicate to the incoming nurse, e.g., the relevant progresses and changes of the patient during the past shift. For example, communicated information may include vitals, sleep, talking, delirium, ventilator weaning, etc. For example, communicated information may include the actions that have been performed and actions that await performing. For example, such actions may include, e.g., medication refill, planning for the day, visits, doctor diagnostics, operations, scans, etc. For example, these may be discussed, say, first in a nurse room, and later in more detail in the patient room.

For example, in the morning, an intensivist and his/her assistants may visit each of the ICU patients. The relevant information may be summarized by the nurse(s) to the doctors, they discuss together the options for actions and treatment, and the doctor may leave instructions to the nurse.

If the patient has to follow a rehabilitation program, the nurses, doctors, speech therapists, physiotherapist, transition carer, patient and family may have to interact and discuss the possible rehabilitation options and their consequences.

In current practice, information is exchanged in these situations by voice, possibly supported by looking at information on a monitor, or on equipment at the bedside, etc. The inventors found that this situation is not ideal. For example, if information is only available on a screen, then the actors need to focus somewhere else, away from the patient. Focus may have to switch back forth multiple times between screen and patient. Such a situation removes focus from the patient, is conducive to errors, and is time consuming. It may create situations in which several people look at the same time at a screen during a discussion, instead of looking at each other or at the patient or the family. The latter is especially important to make the patient and family feel included in the care process.

Another problem is that handover of patient information may occur at places where no screen is available, e.g., in the corridor, around the bed of the patient, etc. Using only voice for information transfer, without the support of a screen increases the likelihood that information gets lost.

Those problems are here described in the ICU context but are recurrent in many other points of the health continuum, from care at home to other departments of the hospital.

### SUMMARY OF THE INVENTION

It would be advantageous to have an improved method for positioning a virtual display for displaying information, in particular patient information. For example, in an embodiment, a rule base may be applied to a viewing context to obtain a position for the virtual display. The viewing context may be determined from the viewer identity and/or patient identity, etc.

Virtual displays make it possible to cause a display to appear at a convenient location. Although this is advantageous, the inventors realized that what is convenient depends on the viewing context. For example, when talking face to face a convenient position for a virtual display may be close to the face of the person with which a viewing person, e.g., a person viewing the virtual display is having a conversation, e.g., at eye-level. But if the viewing person is performing an examination of the body or legs, then it would be inconvenient to display the virtual display near the body or legs, etc.

In an embodiment, a virtual display may be displayed for multiple viewing persons. The virtual display may be positioned at the same apparent position for the multiple viewing persons. This has the advantage that the viewing person can share around the virtual display, and can point at the virtual display. On the other hand, the optimal display position may be different each of the viewing person. For example, this may be the case, when more than 2 people are involved in the conversation, because the roles of the viewing persons in the discussion may be different. Thus in some cases, optimal position is one position for all viewers; in some other contexts it is better to split into 2 or more positions.

In an embodiment, the position of the virtual display may be dependent upon detected emotions of the patient. For example, adapting the position of the virtual display can force the doctors to look near the patient and thus make him feel included.

An aspect of the invention concerns a system for positioning a virtual display for patient information. The system is an electronic system. The method is a computer-implemented method. The electronic device may be a computer, server, or the like.

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium. Another aspect of the presently disclosed subject matter provides a method of making the computer program available for downloading.

### BRIEF DESCRIPTION OF THE DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,
Figure 1a and 1b schematically show an example of a patient and patient bed,
Figure 2 schematically shows an example of an embodiment of a positioning system for a virtual display,
Figure 3a schematically shows an example of an embodiment of a positioning system for a virtual display,
Figure 3b schematically shows an example of an embodiment of a virtual display,
Figure 4a schematically shows an example of an embodiment of a rule base,
Figure 4b schematically shows an example of an embodiment of method of positioning,
Figure 5 schematically shows an example of an embodiment of a virtual display positioning device,
Figure 6 schematically shows an example of an embodiment of a method for positioning a virtual display,
Figure 7a schematically shows a computer readable medium having a writable part comprising a computer program according to an embodiment,
Figure 7b schematically shows a representation of a processor system according to an embodiment.

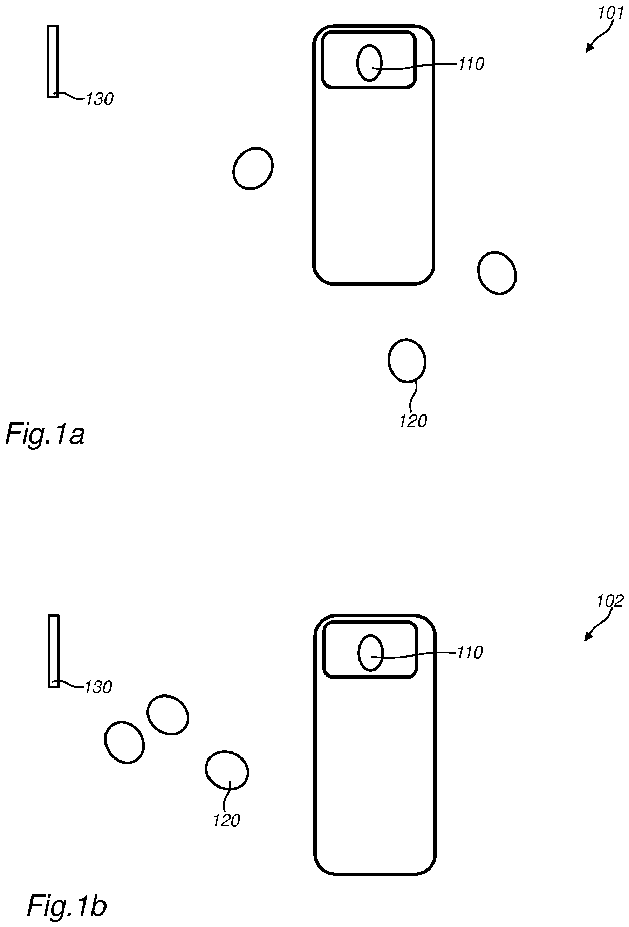

### List of Reference Numerals in figures 1-3b,5, 7a, 7b:

- 101, 102: a hospital room
- 110: a patient
- 120: a person
- 130: a static display
- 200: a hospital room
- 210: a patient
- 221, 222: a viewer person
- 230: a virtual display
- 241: a camera
- 242: a microphone
- 243, 245: an ID-tag
- 244: a motion sensor
- 246: a virtual display device
- 247: a beacon
- 248: smart glasses
- 251: a computer
- 252: a patient information database
- 253: a rule base
- 300: a hospital room
- 310: a patient
- 321-323: a viewer person
- 331-336: a virtual display
- 340: a device
- 350: a display halo
- 351, 352: an information pane

- 500: a system for positioning a virtual display
- 511: a first interface
- 512: a second interface
- 521: a storage interface
- 531: a display interface
- 540: a processor system
- 560: a computer network
- 561: a patient location device
- 562: a viewer person location device
- 563: a projection device
- 564: a sensor

- 1000: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system
- 1100: a device

### DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the presently disclosed subject matter is not limited to the embodiments, as feature described herein or recited in mutually different dependent claims may be combined.

Today through the health continuum, medical information is confined to a static screen. This creates inefficiencies in the workflow and in information communication, as well as impersonal communication as people focus on a screen while talking. **Figure 1a and 1b** schematically show as a top view an example of a patient and patient bed in which no use is made of virtual displays. Figure 1a shows a hospital room 101and a patient 110. One or more persons are in the vicinity of the person, e.g., within speaking distance. Three persons are shown, one of which is marked with reference numeral 120. At some point information from a health data system, e.g., a health data system, needs to be consulted. Figure 1b shows the room 102, wherein the persons have moved to a static display 130 to look at the information.

Note that when going from room 101 to 102 focus is removed from the patient towards the display. This focus may need to change multiple times, e.g., cycling multiple times through hospital room situation 101 and 102. This situation is far from ideal.

In an embodiment these problems are addressed, inter alia, with a virtual display. The virtual display displays context-relevant data about the patient. The virtual display can be viewed by different viewing persons and in different contexts. Thus, the virtual display can be opened through the health continuum, e.g., travels with the patient among different contexts that the patient may encounter in the health continuum, e.g., ranging from ICU to OR, to ward, and so on. Interestingly, the position of the virtual display may be determined dynamically, dependent on factors such as the viewer person's position, the patient's position, interaction among these actors, and topic of discussion. The system may also be applied when a patient consults with a doctor, e.g., a general physician.

The dynamic positioning of the virtual display contributes to a better sharing of information and/or to an optimized workflow, e.g., by providing information at the right place and time, and a better inter-personal communication between care professional, the family and the patient. Better placement of the virtual display also reduces the time that a viewing person needs to change focus; for example, a glance at information may be sufficient and thus intrudes little on the personal communication. Compared to figure 1b, the time to focus back and forth between the display and the patient is reduced significantly. Moreover, by displaying information, say, close to eye-level of a patient, the change in focus is also much smaller and will be noticed much less by the patient.

The information that may be displayed on the virtual display may be context-relevant data about the patient. For example, from information such as the persons that are present, their roles, their schedule, a viewing context may be derived. Interestingly, this may also include the topic of conversation; the latter may be determined using computer implemented speech analysis of a recorded speech. For example, the speech analysis may scan the recording for keywords and display relevant information if such keywords are encountered. For example, if the keywords intravenous infusion or intravenous therapy are recognized, an information panel may be shown next to an intravenous infusion device.

The virtual display may show a summary of clinical data relative to the patient. The content can adapt dynamically to the context, e.g., viewer, time, location, task, topic of discussion, and so on. Such adapting displays are sometimes referred to in the art as dynamic dashboards, known solutions for adapting the displayed information, e.g., known dynamic dashboards, may be adopted in embodiments. Likewise, techniques for displaying of a virtual display, e.g., a display having a position that is not fixed, and can be set by a projecting device, are known and can be applied in an embodiment. Typically, an augmented reality device such an augmented reality headset or smart glasses may be used to display the virtual display. An augmented reality device allows the position of the virtual display to be personalized to the user of the augmented reality device. With a projecting device, there is only one display position which will be the same for all the viewers; a projecting device may still allow a position which is collectively optimized.

A virtual display may be visible to some but not necessarily to all persons present. A virtual display may be generated separately for multiple viewing persons, e.g., for smart glasses of the multiple persons, e.g., as augmented reality; the multiple person may still see the same content and on the same place. This contributes to collaboration on the data.

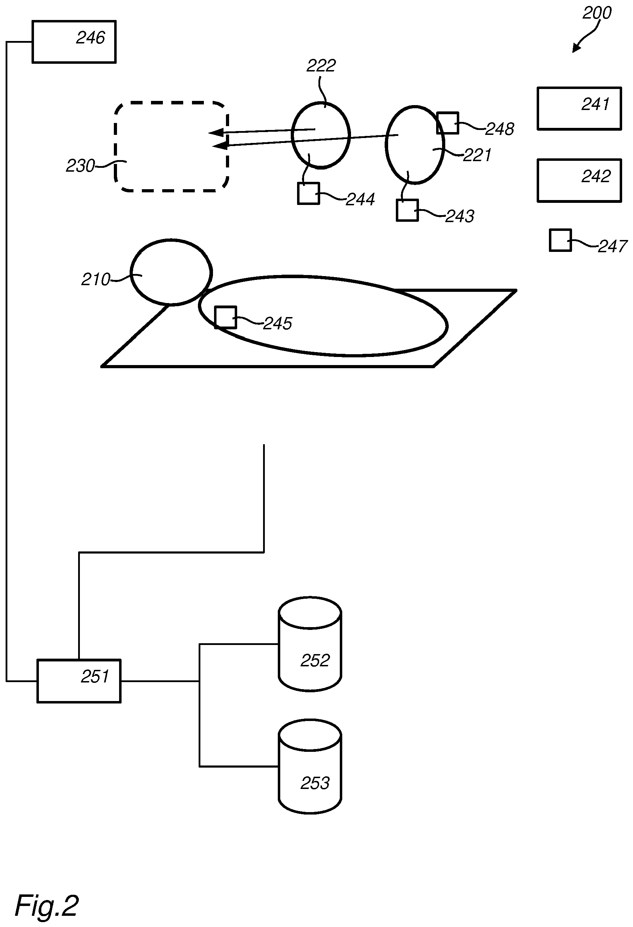

**Figure 2** schematically shows an example of an embodiment of a positioning system 200 for a virtual display. Figure 2 shows a patient 210 and two viewing persons: viewing person 221 and viewing person 222. A viewing person may be a person who needs to consult health information concerning patient 210, e.g., an actor in the health continuum, e.g., a doctor, nurse, speech therapist, and so on. There may be only a single viewing person; there may be more than 2 viewing persons.

Room 200 comprises equipment that may be used to obtain a patient signal and a viewer signal indicating the identity and/or presence of patient 210 and viewer persons 221, 222. For example, one or more ID-tags may be used. Shown in figure 2 are an ID-tag 243 associated to a viewing person, and an ID-tag 245 associated with the patient. For example, the ID-tag may be worn on the body of the respective persons. For example, a camera 241 may be used for face recognition of the persons (patient and/or viewing persons) in room 200. For example, a microphone 242 may be used for voice recognition. Which type of equipment is used to identify the patient and/or viewing person is optional. The respective signals may be received by a computer 251.

Computer 251 typically will not be present in room 200. Computer 251 may be configured to identify patient and viewing persons from the respective signals that indicate them. Note that the signals for patient and viewing person(s) need not be of the same type; for example, patient may use a tag, while viewing persons are recognized by face recognition, or the other way around, etc.

The computer may also determine a patient position and/or viewer position from the patient signal and/or the viewer signal. For example, patient position and/or viewer position may be determined with respect to a fixed point, e.g., the room, a beacon, etc., or relative to each other, or relative to the wearer, etc. A patient position may be determined statically, e.g., from a database which records which patients are located in which room. A location may be determined from an indoor location system. For example, one or more beacons may be placed in or around room 200. Figure 2 shows a beacon 247. For example, a beacon may transmit a room number, which may be forwarded by an ID-tag to computer 251. The strongest beacon signal likely corresponds to nearest beacon, which may be the room in which the ID-tag is currently located. Other known location systems may be used or combined, beacon-triangulation, GPS, Wi-Fi tracking, and so on.

Computer 251 is configured to cause the virtual display to be displayed at least for one viewer person at a particular display position which may be dynamically determined, e.g., also by computer 251. Patient information may be shown on the virtual display. For example, computer 251 may send a display signal that comprises a display location and/or a display content. Figure 2 shows a virtual display device 246. Various options are possible for virtual display device 246. For example, the virtual display may comprise an augmented reality display, a hologram display, a projection display, e.g., a projector, etc. Virtual display device 246 is configured to cause a virtual display to become visible at a specified location and with a specified content to at least one virtual display.

A particular, advantageous choice for the virtual display device are so-called smart glasses, or smart goggles. Figure 2 shows smart glasses 248 used by viewing person 221. For example, the smart glasses may overlay an augmented reality on what the person wearing the glasses sees. In particular, it will appear to the wearer, e.g., person 221, that a virtual display is visible at a particular location with a particular content even though for the other persons in room 200, this is not visible. A common virtual display may be shown to all or a subset of the viewing persons present in the vicinity of the patient; the virtual display may be at the same apparent location. One or more viewing persons may in addition see a private virtual display.

Computer 251 may be configured to determine a viewing context. The viewing context may be used to determine what information should be displayed in the virtual display and/or viewing context may be used to determine the position where the virtual display may be displayed. For example, a viewing context may be determined from the viewer identity and/or patient identity; For example, the viewing context may be structured or unstructured data that contains these and/or other elements.

A rule base may be applied to the viewing context to determine the display content and display location. As pointed out, techniques, known from dynamic dashboards, may be adopted to determine a content. For example, computer 251 may be connected to a patient information database 252, e.g., a health data base. Computer 251 may retrieve information associated with patient 210 from database 252, in particular, context-dependent information and cause the information to be displayed on a virtual display.

A rule base may comprise one or more rules that take one more elements of the viewing context and produce a position as output. For example, computer 251 may be connected to a rule database 253. Computer 251 may retrieve rules and apply them to the viewing context. For example, a rule may also indicate its applicability, e.g., in the form of a confidence value that its output should be selected. For example, for a doctor a different rule may be applicable as for a doctor. This may be implemented by computing a confidence value, e.g., if a value which may be higher if the role of the viewing person corresponds to a role expected by the rule. Rule selection is also possible with confidence values, e.g., using meta-rules.

A rule may apply only if the viewing person is in the vicinity of the patient. For example, the system may be configured that as the viewing person approaches the patient, the virtual display becomes visible to the viewing person. The vicinity may be a threshold, e.g., geographic distance from the patient. For example, a virtual display may be activated when a viewing person enters the vicinity of the patient, e.g., comes within a predetermined or dynamically determined distance of the patient. The virtual display may also be activated by a viewing person, e.g., using a person device, e.g., a tablet, smart glass, or the like, or using image or voice recognition.

Initially, a default position may be chosen for the display position, but as the viewing context comprises more data, a better position can be found. For example, initially, the display may be shown on eye-level of the patient and contain generic information. But speech analysis makes clear that treatment options are being discussed, the display may be moved to a lower level , e.g., to facilitate interpersonal communication, while still allowing access to information at a glance. Instead of a lower level, a side-ways position may be used.

Interestingly, by determining a viewing context, which is dynamic based on the circumstance, a different position may be chosen for a different context. For example, the virtual display may be used by various health professionals, as the patient encounters different persons and settings, e.g., as he/she moves between departments of a hospital, in a consult room, at a fixed position relative to the patient. For example, in a different clinical context, e.g., an OR, different information may be needed than in a ward.

For example, the position may be in a coordinate system, e.g., relative to the viewing person. Preferably, the position is dynamically updated to account for movement of the head and/or body of the viewing person, so that the virtual display appears to be at a stable position.

For example, figure 2 shows a virtual display 230 which is visible both to persons 222 and 221. For example, both persons may wear smart-glasses that are each configured to display the virtual display to the viewing persons.

The position of the virtual display may be adjusted by sending an adjustment signal from a viewing person, e.g., to computer 251. Computer 251 may adjust the displaying position as indicated by the adjustment signal. For example, one or both of the viewing persons may be equipped with a motion sensor 244. The motion signal may be used to indicate an adjustment, e.g., by swiping the virtual display away or to a different location. The motion sensor may be used as part of the viewing context, e.g., to indicate a type of interaction that is currently ongoing. Adjustment may be made using image recognition through a camera, voice recognition, a personal device, and so on.

Note that many elements of embodiments are optional and/or may be varied. For example, a virtual display, e.g., a virtual health data display may use AR, projection, holograms, smart-glasses, and so-on. For example, the system may detect the persons present, their role, position, tasks and/or the topic of their discussion, e.g., through tags, camera recognition, speech analysis, and so on. Moreover, the rules for determining the position of the virtual display from the context may be varied in a number of ways.

Note, that the system may also decide that in some circumstances a display on a regular static display is a good option. For example, if the system determines that a viewing person is near a static screen and not near a patient than it may show the patient information on the static screen. The nearness may be determined by a distance, e.g., at least 1 or 2 meters away, from patient, and at least within 1 meter of a static screen.

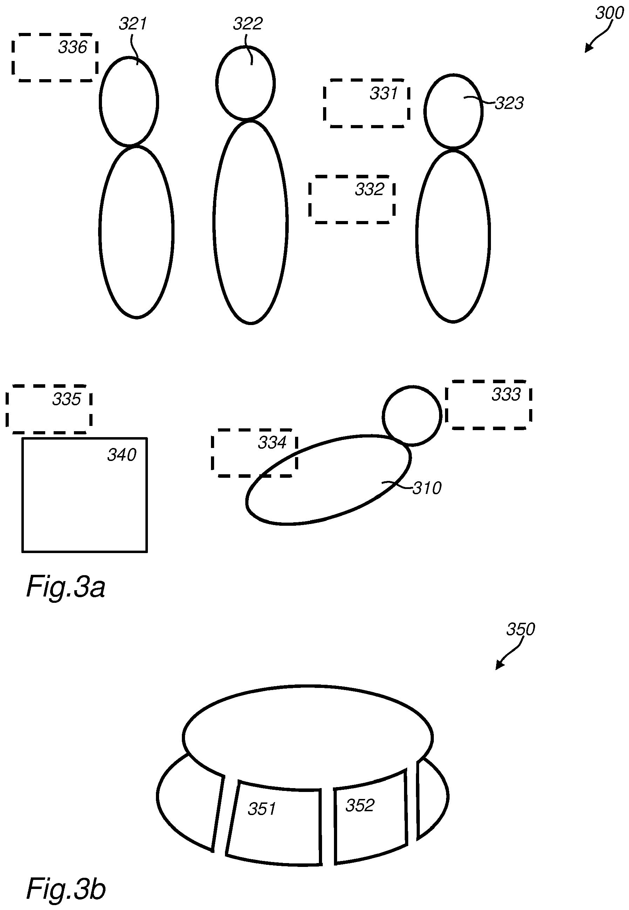

**Figure 3a** schematically shows an example of an embodiment of a positioning system for a virtual display. Figure 3 shows a hospital room 300 including a patient 310 and multiple viewer persons: persons 321, 322 and 323. Multiple examples of a virtual display positions are exemplified in figure 3. Typically, not all position will be used at the same time, and some positions may not be used at all.

Virtual display 333 is located at an eye level of the patient. In this case, the virtual display is also shown near the patient's eyes, e.g., next to his/her head. A virtual display may be located below an eye level of the patient and viewing person. Note that in the figures, patients are drawn in a reclined position, but this is not necessary. Embodiment may also be applied to sitting or standing patients.

Virtual display 335 is located relative to a device 340 located in the vicinity of the patient. For example, if it is detected that the discussion is related to a device, e.g., a medical device in room 300, information relevant to the device may be shown. For example, if speech analysis, or eye direction analysis shows that a blood pressure measuring device is discussed, then a history of previously measured blood pressure may be displayed on the virtual display.

Virtual display 334 is located at a specific body part of the patient. For example, if a leg of the patient is under discussion, or under examination, then information from the medical history relevant to the leg may be displayed close to the leg. For a care professional who is examining the leg it is of advantage that the virtual display is positioned close to the leg under examination, e.g., if the virtual display shows information that is relevant to the examination, e.g., an x-ray image of the leg. For other people present in the room who are not close to the leg, e.g., nurse, other doctor, patient, family, etc., a virtual display may be set elsewhere, e.g., at eye level. This avoids all of the people present having to bend over the leg, Moreover, visibility would not be not optimal for most people present. Thus the same display may be displayed at multiple positions, e.g., optimized for different people. This is referred to as a split virtual display. Note that fewer positions may be selected than that there are viewing persons present. For example, the viewing persons close to the leg may use a one display positions, while the other viewing persons may use a central position. This has the advantage that a sense of shared ownership is retained, while at the same time viewing is optimized. Detecting an activity, such as examining a leg, may be done by image analysis, e.g., by applying a machine learnable model, such as a neural network.

Virtual display 332 is located at a location between multiple viewing persons and/or patient, e.g., a geometric center. For example, if multiple persons are discussing some topic, e.g., medical images, e.g., x-ray images, then these images may be shown at a central location.

There are several position options for a group of viewing persons. For example, the position may be at a central location between all viewers below/near eye level. This option may be used, e.g., for viewers involved in a discussion, e.g., patients and family and doctors talking together. Another option is at a location central between all but with an offset towards the viewers who are most active in the discussion. For example, this may be used when two viewers are actively looking at the data and discussing it, e.g., one doctor and a patient, while the others are followers of the discussion, e.g., nurse and family and junior doctor. The virtual display position may be near the most active viewers, but still have the display more or less between everybody to give the cohesive/inclusive feeling. In an embodiment, the position or positions where a virtual display is displayed, may be changed dynamically, e.g., depending on the relative activity of the viewers

In an embodiment, the virtual display is positioned between the actors of a discussion and just below eye level, so that the actors can look at each other. When it is detected that a specific data/image is addressed in the discussion, the virtual display may be raised to eye level of the actors, e.g., showing the specific data/image

Virtual display 331 is located at an average eye level of multiple viewing persons; the location may be at an average eye level of multiple viewing persons and patient.

The virtual displays above may be visible to one or to more than one or to all of the viewing persons. The patient may or may not be a viewing person himself. The virtual displays may or may not be visible to the patient. The system may be configured with shared and private virtual displays. For example, virtual display 336 is only visible to person 321. For example, virtual display 336 may comprise specialized information that will not be useful to other persons in the room. At the same time one more of displays 331-335 may be visible to person 321. Various combinations are possible; e.g., one or more displays being visible to one or some person, one or more displays being visible to all persons, etc. For example, display 336 may be displayed only to person 321, but not to the other viewing persons and the patient. For example, a virtual display may be one common dashboard visible by all actors, and next to it the individual actors can each have an extra dashboard containing their respective key information.

Eye level may be detected using a camera using an eye recognition algorithm, e.g., a facial landmark algorithm.

In an embodiment, the system, e.g., computer 251 may receive an input from the patient and the virtual display may be adapted thereupon. This may be used by the patient to communicate. Communication may be yes/no answers, selection from a list of phrases, and so on. For example, the input may be given with a sensor, motion sensor, camera, smart phone and so on. For example, the display position may be at the hand and/or eye level of the patient to facilitate the use of the virtual display as a communication means. For example, a doctor and nurse in the patient room may try to communicate with a patient which has impaired speech.

In an embodiment, computer 251 may determine an emotional parameter, e.g., the emotional load of the discussion. This may be done using, e.g., speech analysis, or the so-called 'emotional response of the patient', e.g., via physiological sensing such as heart rate, skin conductivity, or facial expression analysis of the patient. If an increased emotional load is detected, e.g., above a threshold, this may trigger to position the display moved next to the patient so that the doctors need to look at the patient and involve them.

The position of the virtual display may be adjusted manually in an embodiment. In an example, an anesthesiologist is working in the OR. For example, adjustment may be done with a gesture, which may be detected using a motion sensor or camera. The system may detect that he was preparing the patient, so that the virtual display may be placed next to the body part that he was inspecting. For example, this may be according to a rule. The anesthesiologist can move the virtual display to a specific place that he finds more convenient, e.g., on a separation wall in the OR.

**Figure 3b** schematically shows an example of an embodiment of a virtual display. The virtual display may have many shapes, e.g., shaped as a static display. But other shapes, e.g., 3d rather than 2d shapes are also possible. For example, figure 3b shows a display halo 350, e.g., a cylindrical shape, e.g., a cylinder, frustum, a conical frustum, etc. For example, on the cylindrical shape information may be shown. In figure 3b, the halo comprises multiple information panes arranged along the cylindrical shape. Shown are two panes: panes 351 and 352; there may be more or fewer panes.

For example, pane 351 may show a history of blood pressure, while pane 352 may show name and age, etc. In an embodiment, a viewing person may move the cylindrical shape, e.g., by swiping or the like to make other information panes visible.

Depending on the use case, the required information and convenient position may change. For example, one may prefer to have the information:
- on a static screen for one medical actor without interaction with a device or patient, or
- in between several actors discussing for information sharing between medical actors and/or patient and/or family, or
- near the eye level of the patient for discussion involving the patient, or
- on top of a device for discussion relating to a device e.g., medication dispensing, or
- on top of a specific body part of the patient for discussion relating to a body part of the patient or for examination/operation on a body part of the patient or for anesthesiologist on a body part which is away from the operated area.

This may be achieved by a suitable rule base, for example, a rule may determine or assess the antecedents of multiple rules. For example, determine a 15% likelihood that a body part of the patient of is being examined, but a 65% likelihood that this is a conversation between several actors, e.g., viewing persons. Accordingly, the latter rule may be selected, and the virtual display may be positioned in between the persons. To evaluate an antecedent of a rule, a model may be used, that takes as input sensor data, e.g., camera, microphone, motion information, etc. The model may be hand crafted but may also be a machine learning model, e.g., an SVM, neural net, or the like.

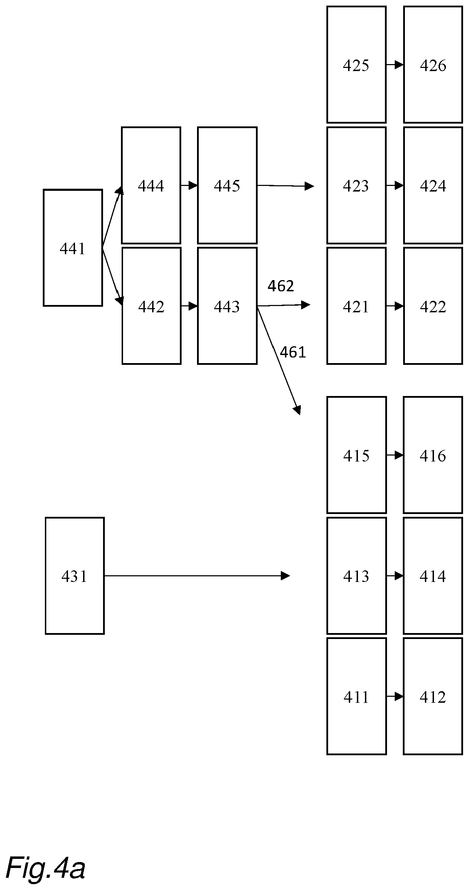

**Figure 4a** schematically shows an example of an embodiment of a rule base. Shown in figure 4a is a decision on the number of actors:
- 431: One actor
- 441: Several actors

In case of multiple several actors, the system may determine if the actors talk together or if one actor is performing a task:
- 442: One actor is performing a task
- 444: Actors are talking together

A corresponding decision may then be to have one common or shared virtual display or the have multiple, e.g., split, virtual displays. For example, one may have:
- 443: Split virtual display
- 445: One common virtual display

In case of split virtual displays, the arrows at 461, 462 indicate that different rules may be followed for the two or more virtual displays. Below multiple rules are shown which may be followed in either circumstance. For example,
411 Examining/entering data in dashboard
412 Static dashboard on screen or virtual display at eyes level
   and/or
413 Examining patient
414 virtual display on patient, outside (e.g., 20 cm) of examination area
   and/or
415 Using device
416 virtual display next to device UI
   and/or
421 Knowledge not yet in dashboard (e.g., doctor gives treatment recommendation)
422 virtual display in the center of the actors, below eye level
   and/or
423 Knowledge in dashboard (e.g., nurse gives patient summary)
424 virtual display in the center of the actors, at eye level
   and/or
425 Non-factual/emotional discussion, e.g., doctor explains risks to family)
426 virtual display in a parking position, e.g., away from field of vision of actors

The above example rule base may be modified in various ways. For example, a split virtual display may be selected if one actor is performing a task, but a this may also be selected, e.g., if two or more actors are more involved in the discussion than the other people. The later may be detected via, e.g., speech analysis, gaze analysis, and so one.

The example rule base above may regarded as a hierarchical rule base. This is not necessary, but allows high specificity which high flexibility. For example, in rule 431/ 441 it is determined how many actors there are, e.g., how many viewing persons. The outcome of the rule determines which further rule base is applied . A hierarchical rule set may be flattened by incorporating the higher-level antecedents in in lower level rules. But in its hierarchical form, the rule set is easier to understand and to modify.

For example, for an ICU/OR handover, around the patient bed, the ICU nurses are summarizing the patient data to the OR nurse and the anesthesiologist. Rule 423/423 may apply, and the virtual display may be positioned at the average eye level of the actors, e.g., at the geometric center of the shape formed by the actors.

For example, in the OR, in this case where the patient is going to receive brain surgery, the surgeon is positioned at the head side and the anesthesiologist at the feet side. Rule 413/413 may apply, and the virtual display may be positioned for the anesthesiologist against the separation wall facing him, while it is on the other side for the surgeon. The separation wall may be at the patent neck's level.

For example, clinical information about the patient are displayed for the doctor and nurse in an additional virtual display, e.g., located next to the patient. Rule 413/413 may also apply to one or both of split virtual displays. Possibly the doctor and nurse can move the additional virtual display with a gesture to, e.g., the chest of the patient.

In this example, the fourth column indicates a condition that needs to be satisfied and the fifth column indicates an action to be taken for the virtual display. The rows in figure 4a may indicate a dependency between rules, antecedents and position decisions.

Many elements can be used in rules, e.g., in their antecedents. For example, these elements may be collected in a viewing context. For example, the viewing context may be determined from one or more of
- a position of a viewer person relative to the patient,
- a position of two or more viewer persons relative to each other,
- an interaction between multiple viewing persons,
- patient information retrieved for the patient,
- a location of the patient,
- a role and/or task of the viewer person,
- a topic detected in a conversion among one or more viewing persons and/or the patient as determined from a computer implemented speech analysis.

The viewing context may comprise information that indicates one or more of the above information; the viewing context may also comprise information that has been further processed, e.g., that the viewing context is an emotional discussion. Whether or not an emotional discussion may be computed from another rule or model, e.g., a machine learnable mode, etc.

For another example that can be accommodated with a rule base consider a situation in which an intensivist, a speech therapist, the patient and his wife are discussing intubation options for transfer out of the ICU. The Virtual display with the main information may be positioned at the center of the actors, while the individual actors can see their specific information on an additional virtual dashboard closer to them. When initially the discussion is about future options the central virtual display may be positioned below eye level so that people can look at each other while talking and glimpse occasionally at the common virtual display or their personal virtual display; for example this may apply rule 421/422 and rule 411/412 for split virtual displays. Later in the discussion, the doctor may explain the tracheal suction procedure, the virtual display may be raised to eye level so that everyone can follow the illustrative demonstration displayed; this may apply rule 423/424.

Using a rule base is particular flexible yet powerful way to adjust the system to the requirements in a particular setting.

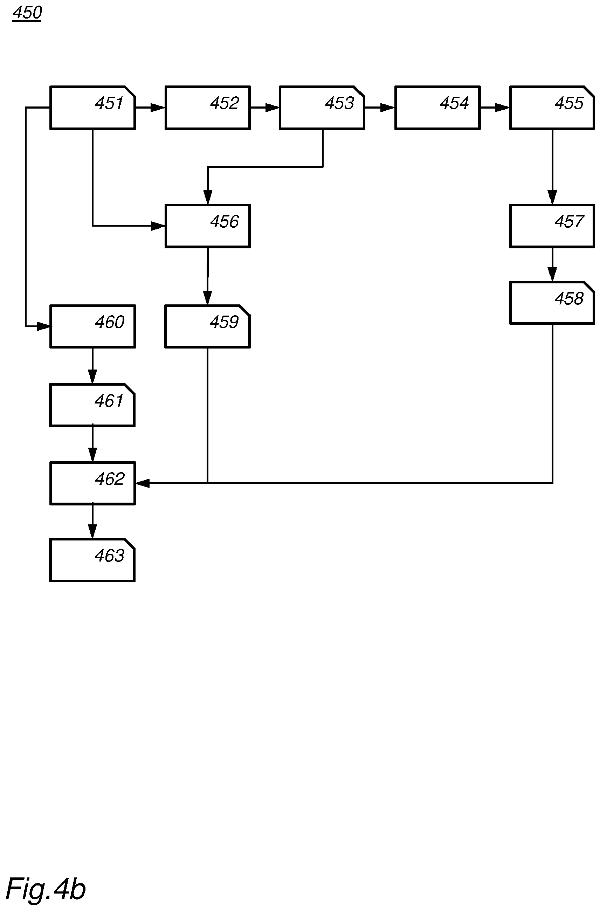

**Figure 4b** schematically shows an example of an embodiment of method 450 of positioning a virtual display. A possible workflow that may be followed is illustrated in figure 4b.

Shown in figure 451 is input data 451. The input data may include sensor data such as include camera, microphone, motion sensor data. The input data may also include location and identity data of viewing persons and a patients. The input data may be combined in a viewing context. The input data may be processed by one more models 452, in particular machine learning models. The models may identify various aspects in the data, e.g., actions taken, e.g., examination of a body part, devices that are operated, e.g., topics that are discussed and so on. Models 452 may also perform recognition of the persons in view. The results may be recognition data 453 which may also be included in the viewing context. Rule base 454 may be applied to the viewing context, e.g., on input data and/or on recognition data. The rules may be hierarchical or not. For example, a set of position data 455 of (confidence, position) pairs may be result. A final position selection is made in position selection 457, e.g., by selecting the highest confidence value. This result in virtual display position data 458. Rule base 454 may also directly produce virtual display position data 458.

Input data 451 and recognition data 453 may also be processed by a content selection algorithm 456. Content selection 456 may select and retrieve content data 459 that is relevant for the context. For example, if blood pressure is discussed, then blood pressure values may be retrieved. Based on the input data 451, a location algorithm 460 may determine the current location and/or position of the actors, e.g., viewing person, patient, in the room. This may be actor position data 461. Possibly also other items may be located, e.g., a device in the room.

Finally, the actor positioning data 461, content data 459 and virtual display positioning data from 458 may be combined into a display signal by display driver algorithm 462, resulting in display signal 463. Display signal 463 may be configured for, e.g., a projector or for smart glasses.

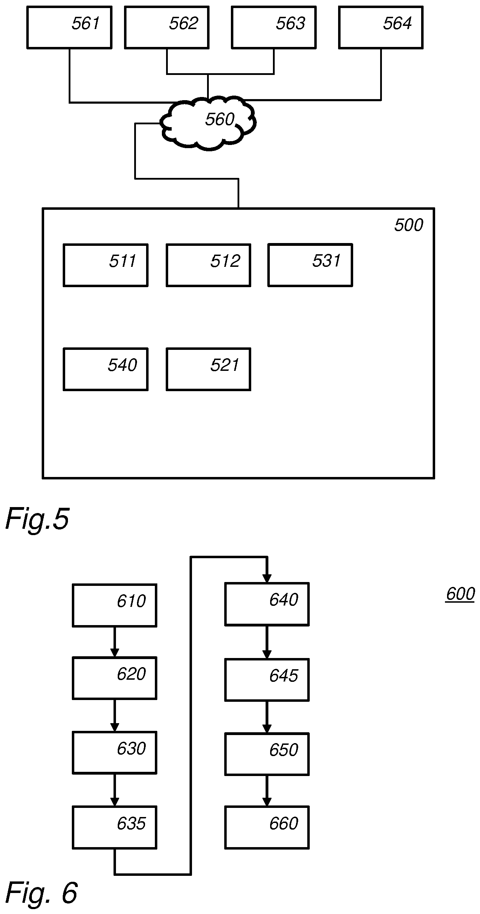

**Figure 5** schematically shows an example of an embodiment of a virtual display positioning device 500. Virtual display positioning device 500 is an example of a virtual display positioning system. In the latter case, some elements of the device may be implemented in a distributive fashion, e.g., using cloud computing.

Device 500 comprises a first interface 511 configured to receive a patient signal that indicates a patient identity, and a second interface 512 configured to receive a viewer signal indicating the presence of at least one viewer person located in the vicinity of the patient. Device 500 may also comprise a storage interface 521 configured to retrieve patient information from a health data system, e.g., a patient information database, through the patient identity, and a display interface 531 configured for sending a display signal to cause a virtual display to be displayed for the viewer person at the display position and to display the patient information on the virtual display. Rule base and patient database may be part of device 500 or may be located elsewhere, e.g., in the cloud.

For example, the interfaces may connect to a computer network 560. The computer network 560 may be partially wired and partially wireless. Computer network 560 may be LAN or the internet, etc.

For example, device 500 may receive signal through network 560 from a patient location device 561, a viewer person location device 562, e.g., an ID-tag, a projection device 563, e.g., smart glasses, and a sensor 564, e.g., a camera, microphone or motion sensors. Other examples, e.g., as discussed herein may also be used. Location device 561 and 562 may also be configured to establish the identity of their patient and/or viewing person. For example, location device 561 and/or 562 may be an Id-tag, smart phone, smart glasses, etc. Location and/or identify may also be established from face or voice recognition.

Device 500 comprises a processor system 540. For example, processor system 540 may comprise one or more microprocessors and a memory storing computer instructions for execution by the one or more microprocessor. Processor system 540 may be configured for
- determining a viewer identity for the at least one viewer person from the viewer signal,
- determining a patient position and/or viewer position from the patient signal and/or the viewer signal
- applying a rule base to a viewing context determined from the viewer identity and/or patient identity, thus determining a display position relative to the patient and/or viewer position,

The various devices of figure 5 may communicate with each other over a computer network 560. The computer network may be an internet, an intranet, a LAN, a WLAN, etc. Computer network 560 may be the Internet. The computer network may be wholly or partly wired, and/or wholly or partly wireless. For example, the computer network may comprise Ethernet connections. For example, the computer network may comprise wireless connections, such as Wi-Fi, ZigBee, and the like. The devices comprise a connection interface which is arranged to communicate with other devices of figure 5 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

The execution of a positioning method may be implemented in a processor circuit, examples of which are shown herein. The processor system may comprise functional units to implement various features of embodiments. For example, a functional units may be wholly or partially implemented in computer instructions that are stored at device 500, e.g., in an electronic memory of device 500, and are executable by a microprocessor of device 500. In hybrid embodiments, functional units are implemented partially in hardware, e.g., as coprocessors, and partially in software stored and executed on device 500.

In the various embodiments of positioning devices and/or systems, a communication interface may be selected from various alternatives. For example, the interface may be a network interface to a local or wide area network, e.g., the Internet, a storage interface to an internal or external data storage, a keyboard, an application interface (API), etc. The positioning device may have a user interface, which may include well-known elements such as one or more buttons, a keyboard, display, touch screen, etc. Storage may be implemented as an electronic memory, say a flash memory, or magnetic memory, say hard disk or the like, or optical memory, e.g., a DVD. Storage may comprise multiple discrete memories together making up the storage. Storage may comprise a temporary memory, say a RAM

Typically, the positioning device or system comprise a microprocessor which executes appropriate software stored thereon; for example, that software may have been downloaded and/or stored in a corresponding memory, e.g., a volatile memory such as RAM or a non-volatile memory such as Flash. The projecting device, tags, smart glasses, sensors etc., may also be equipped with microprocessors and memories. Alternatively, devices may, in whole or in part, be implemented in programmable logic, e.g., as field-programmable gate array (FPGA), or may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc.

A processor circuit may be implemented in a distributed fashion, e.g., as multiple sub-processor circuits. A storage may be distributed over multiple distributed sub-storages. Part or all of the memory may be an electronic memory, magnetic memory, etc. For example, the storage may have volatile and a non-volatile part. Part of the storage may be read-only.

**Figure 6** schematically shows an example of an embodiment of a method for positioning a virtual display 600. Method 600 may comprise
- receiving (610) a patient signal that indicates a patient identity,
- retrieving (620) patient information from a patient information database through the patient identity,
- receiving (630) a viewer signal indicating the presence of at least one viewer person located in the vicinity of the patient and determining (635) a viewer identity for the at least one viewer person from the viewer signal,
- determining (640) a patient position and/or viewer position from the patient signal and/or the viewer signal
- applying (650) a rule base to a viewing context determined (645) from the viewer identity and/or patient identity, thus determining a display position relative to the patient and/or viewer position,
- sending (660) a display signal to cause a virtual display to be displayed for the viewer person at the display position and to display the patient information on the virtual display.

Many different ways of executing the method are possible, as will be apparent to a person skilled in the art. For example, the steps can be performed in the shown order, but the order of the steps may also be varied, or some steps may be executed in parallel. Moreover, in between steps other method steps may be inserted. The inserted steps may represent refinements of the method such as described herein or may be unrelated to the method. For example, some steps may be executed, at least partially, in parallel. Moreover, a given step may not have finished completely before a next step is started.

Embodiments of the method may be executed using software, which comprises instructions for causing a processor system to perform method 600. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

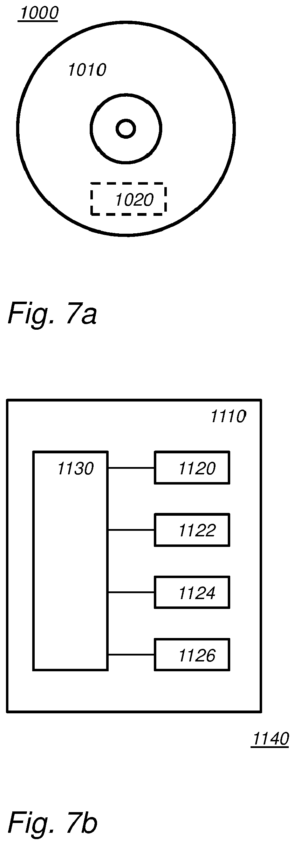

**Figure 7a** shows a computer readable medium 1000 having a writable part 1010 comprising a computer program 1020, the computer program 1020 comprising instructions for causing a processor system to perform a positioning method for a virtual display according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said positioning method.

**Figure 7b** shows in a schematic representation of a processor system 1140 according to an embodiment of a positioning system or device. The processor system comprises one or more integrated circuits 1110. The architecture of the one or more integrated circuits 1110 is schematically shown in Figure 7b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the positioning device may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While device 1100 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 1100 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A computer implemented method (600) for positioning a virtual display for patient information, comprising
- receiving (610) a patient signal that indicates a patient identity,
- retrieving (620) patient information from a patient information database through the patient identity,
- receiving (630) a viewer signal indicating the presence of at least one viewer person located in the vicinity of the patient and determining (635) a viewer identity for the at least one viewer person from the viewer signal,
- determining (640) a patient position and/or viewer position from the patient signal and/or the viewer signal
- applying (650) a rule base to a viewing context determined (645) from the viewer identity and/or patient identity, thus determining a display position relative to the patient and/or viewer position,
- sending (660) a display signal to cause a virtual display to be displayed for the viewer person at the display position and to display the patient information on the virtual display.

2. A computer implemented method for positioning a virtual display for a patient as in Claim 1, wherein the display position is determined differently for a different viewer context.

3. A computer implemented method for positioning a virtual display for a patient as in any one of the preceding claims, wherein the display position is determined to be one of:
- at an eye level of the patient,
- below an eye level of the patient and viewing person,
- relative to a device located in the vicinity of the patient,
- at a specific body part of the patient,
- at a location between multiple viewing persons and/or patient, e.g., a geometric center,
- at an average eye level of multiple viewing persons and/or patient, or
- below an average eye level of multiple viewing persons and/or patient.

4. A computer implemented method for positioning a virtual display for a patient as in any one of the preceding claims, comprising causing the virtual display to be displayed to more than one of the patient and one or more viewer persons.

5. A computer implemented method for positioning a virtual display for a patient as in claim 4, wherein, a further virtual display is caused to be displayed only to some but not all of the one or more viewing persons and the patient.

6. A computer implemented method for positioning a virtual display for a patient as in any one of the preceding claims, wherein the virtual display and a further virtual display are caused to be displayed to the same viewing person.

7. A computer implemented method for positioning a virtual display for a patient as in any one of claims 4-6, wherein the display position is at the hand and/or eye level of the patient, the method comprising receiving an input from the patient and adapting the virtual display based upon the input.

8. A computer implemented method for positioning a virtual display for a patient as in any one of the preceding claims, comprising receiving an adjustment signal from a viewing person and adjusting the displaying position as indicated by the adjustment signal.

9. A computer implemented method for positioning a virtual display for a patient as in any one of the preceding claims, wherein a rule from the rule base is arranged to take one or more elements of the viewing context as input and to produce a relative display position.

10. A computer implemented method for positioning a virtual display for a patient as in any one of the preceding claims, wherein the rule base is hierarchical wherein a higher-level rule determines which lower level rule is applied, e.g., a higher-level rule determining the number of viewing persons.

11. A computer implemented method for positioning a virtual display for a patient as in any one of the preceding claims, wherein the displayed patient information and/or the viewing context are determined from one or more of
- a position of a viewer person relative to the patient,
- a position of two or more viewer persons relative to each other,
- an interaction between multiple viewing persons,
- patient information retrieved for the patient,
- a location of the patient,
- a role and/or task of the viewer person,
- a topic detected in a conversion among one or more viewing persons and/or the patient as determined from a computer implemented speech analysis.

12. A computer implemented method for positioning a virtual display for a patient as in any one of the preceding claims, comprising determining a viewing person is near a static screen and not near a patient and showing the patient information on the static screen.

13. A computer implemented method for positioning a virtual display for a patient as in any one of the preceding claims, wherein the virtual display comprises an augmented reality display, a hologram, a projection.

14. A transitory or non-transitory computer readable medium (1000) comprising data (1020) representing instructions, which when executed by a processor system, cause the processor system to perform a method according to any one of claims 1-13.

15. A system (500) for positioning a virtual display for patient information, comprising
- a first interface (511) configured to receive a patient signal that indicates a patient identity,
- a second interface (512) configured to receive a viewer signal indicating the presence of at least one viewer person located in the vicinity of the patient
- a storage interface (521) configured to retrieve patient information from a patient information database through the patient identity,
- a processor system configured for
- determining a viewer identity for the at least one viewer person from the viewer signal,
- determining a patient position and/or viewer position from the patient signal and/or the viewer signal
- applying a rule base to a viewing context determined from the viewer identity and/or patient identity, thus determining a display position relative to the patient and/or viewer position,
- sending a display signal to cause a virtual display to be displayed for the viewer person at the display position and to display the patient information on the virtual display.
